# EUROPÄISCHE PATENTANMELDUNG

(11) **EP 1 344 501 A1**
(43) Veröffentlichungstag der Anmeldung: **17.09.2003**
(21) Anmeldenummer: 02027503.8
(22) Anmeldetag: 05.12.2002
(51) Int. Cl.: A61D 1/00, A61M 5/34

(54) **Veterinärspritze mit einem Kanülenansatz nach Luer-Lock**

(30) Priorität: 20.12.2001 DE 20120662 U
(71) Anmelder: Henke-Sass, Wolf GmbH, D-78532 Tuttlingen (DE)
(72) Erfinder: Schreijäg, Karl, 78628 Rottweil (DE)
(74) Vertreter: Fehners, Klaus Friedrich, Dipl.-Ing., Dipl.-Wirtsch.-Ing.

(57) **Zusammenfassung**

Es wird eine Veterinärspritze mit einem am ausspritzseitigen Ende (1) eines Spritzenzylinders (2) vorgesehenen Ansatz (4) für Kanülen nach Luer-Lock vorgeschlagen, wobei der Ansatz (4) in seinem vorderen, der aufzusetzenden Kanüle (6) zugewandten Bereich als Hohlzylinder (14) ausgebildet ist, dessen Längsachse mit der Längsachse des Spritzenzylinders (2) der Veterinärspritze (3) fluchtet, und der auf seiner Innenwand (18) ein Gewinde (19) aufweist, das zur Aufnahme eines Ansatzendes (5) einer Kanüle (6) mit entsprechend ausgebildetem Gegengewinde (20) vorgesehen ist, und wobei in dem Innenraum des Hohlzylinders (14) ein, einen gegenüber dem Durchmesser des Innenraumes des Hohlzylinders (14) geringeren Außendurchmesser aufweisender Hohlzapfen (7) angeordnet ist, der den Ausspritzkanal (8) für das Medikament aufweist und in das Ansatzende (5) der aufgesetzten Kanüle (6) einsteht, wobei der Hohlzylinder (14) gegenüber dem Hohlzapfen (7) um seine Längsachse drehbar ist.

## Beschreibung

Die Erfindung bezieht sich auf eine Veterinärspritze mit einem am ausspritzseitigen Ende eines Spritzenzylinders vorgesehenen Ansatz für Kanülen nach Luer-Lock, wobei der Ansatz in seinem vorderen, der aufzusetzenden Kanüle zugewandten Bereich als Hohlzylinder ausgebildet ist, dessen Längsachse mit der Längsachse des Spritzenzylinders der Veterinärspritze fluchtet, und der auf seiner Innenwand ein Gewinde aufweist, das zur Aufnahme eines Ansatzendes einer Kanüle mit entsprechend ausgebildetem Gegengewinde vorgesehen ist, und wobei in dem Innenraum des Hohlzylinders ein, einen gegenüber dem Durchmesser des Innenraumes des Hohlzylinders geringeren Außendurchmesser aufweisender Hohlzapfen angeordnet ist, der den Ausspritzkanal für das Medikament aufweist und in das Ansatzende der aufgesetzten Kanüle einsteht.

Diese bekannten Luer-Lock-Veterinärspritzen zeichnen sich durch ihre verhältnismäßig große Unempfindlichkeit insbesondere bei der Durchführung von Reihenimpfungen aus, sie halten großen Belastungen Stand. Dies insbesondere durch die Verschraubung der Kanüle an der Veterinärspritze mittels der bekannten Luer-Lock-Verbindung und auch durch die einwandfreie Dichtheit der Verbindung. Die für den Einsatz der Veterinärspritze gewünschte Stabilität der Luer-Lock-Verbindung kann jedoch bei der Demontage der Kanüle von der Veterinärspritze, insbesondere nach Verarbeitung öliger Substanzen, Schwierigkeiten bereiten, denn das Abnehmen der Kanüle vom Spritzenzylinder ist meist nicht mehr von Hand möglich, sondern nur noch unter Zuhilfenahme von Werkzeugen. Dabei besteht die Gefahr, zum Beispiel durch Abrutschen, die Kanüle zu beschädigen, beispielsweise zu verbiegen, oder sich auch selbst zu verletzen.

Aufgabe der Erfindung ist es daher, eine möglichst einfache und gefahrlose Bedienung, insbesondere eine leichte Demontage der Kanüle zu erreichen.

Dazu ist bei der eingangs beschriebenen Veterinärspritze erfindungsgemäß der Hohlzylinder gegenüber dem Hohlzapfen um seine Längsachse drehbar.
Durch diese Ausbildung des Ansatzes mit der Möglichkeit, den das Gewinde zum Einschrauben des Kanülenansatzes aufweisenden Hohlzylinder gegenüber dem in den Kanülenansatz einstehenden Hohlzapfen andererseits zu verdrehen, kann die Kanüle in der Weise an der Veterinärspritze befestigt werden, daß sie lediglich auf den, vorteilhafterweise gegenüber dem Hohlzylinder etwas weiter nach außen vorstehenden Hohlzapfen aufgesteckt wird und nun nicht selbst in das im Hohlzylinder des Ansatzes befindliche Gewinde eingeschraubt, sondern vielmehr statt dessen der Hohlzylinder des Ansatzes gedreht wird, wodurch die Kanüle fest und dicht auf den Hohlzapfen des Ansatzes aufgezogen wird.

Diese erfindungsgemäße Lösung bietet also gleich in mehrerlei Hinsicht Vorteile, nämlich zum einen muß nicht mehr die Kanüle selbst gedreht werden, so daß die Gefahr, sie zu beschädigen oder sich zu verletzen, vermieden wird. Zum zweiten bietet der drehbare Hohlzylinder gegenüber der Kanüle die günstigere Bedienungsmöglichkeit für die Luer-Lock-Verbindung, weil nämlich der Hohlzylinder einen größeren Durchmesser als die Kanüle bzw. deren Ansatz aufweist und folglich ein deutlich größeres Drehmoment bei der Montage aufgebracht werden kann, so daß insbesondere bei der Demontage der Kanüle, insbesondere bei festsitzender Kanüle, die Verbindung zwischen ihr und dem Ansatz noch von Hand gelöst werden kann. Zum dritten kann die Position der Kanüle, die ja durch die abgeschrägte Spitze ihrer Nadel bestimmt ist, gegenüber der Veterinärspritze frei gewählt werden, da sie in der gewünschten Stellung aufgesteckt und anschließend, ohne selbst verdreht zu werden, auf den Hohlzapfen aufgezogen werden kann.

In vorteilhafter Weiterbildung dieser Lösung ist der Hohlzapfen mit einem Sockel am Ende des Spritzenzylinders befestigt, der zylindrisch ausgebildet ist und auf seiner Außenwand einen über deren Umfang verlaufende Nut aufweist, in die ein Sprengring eingesetzt ist, und wobei der Hohlzylinder in Richtung zum Spritzenzylinder verlängert ist und mit einer glattflächigen Innenwand auf der Außenwand des Sockels des Hohlzapfens aufliegt und die Innenwand eine deren Nut in der Außenwand des Hohlzapfens genau gegenüberliegende Gegennut aufweist, in die der Sprengring ebenfalls einsteht.

Diese besondere Ausbildung des Ansatzes gewährleistet, daß der drehbare Hohlzylinder unverlierbar mit dem fest am ausspritzseitigen Ende des Spritzenzylinders bzw. der Veterinärspritze befestigten Hohlzylinder verbunden ist.

Um den Sitz des Hohlzylinders auf dem Hohlzapfen besonders stabil auszubilden, weisen der Sockel des Hohlzapfens einerseits und der Hohlzylinder in seinem verlängerten Bereich andererseits größere Außen- bzw. Innendurchmesser auf, als in dem für den Ansatz der Kanüle vorgesehen Bereich.

Damit der Ansatz gegenüber der an ihm zu befestigenden Kanüle bzw. deren Ansatzende eine gefällige Formgebung aufweist, ist die Außenwand des Hohlzylinders in ihrem für den Ansatz der Kanüle vorgesehenen Bereich kegelförmig ausgebildet.

Um die Handhabung, also insbesondere die Kraftübertragung auf den Hohlzylinder zu erleichtern, weist der Hohlzylinder eine Außenfläche auf, die profilierte Grifflächen trägt, wobei insbesondere die Außenfläche auch besonders ausgestaltete Griffmulden aufweisen kann.

Die Erfindung wird nachfolgend unter Bezugnahme auf die Zeichnung beispielhalber noch näher erläutert. Es zeigen:

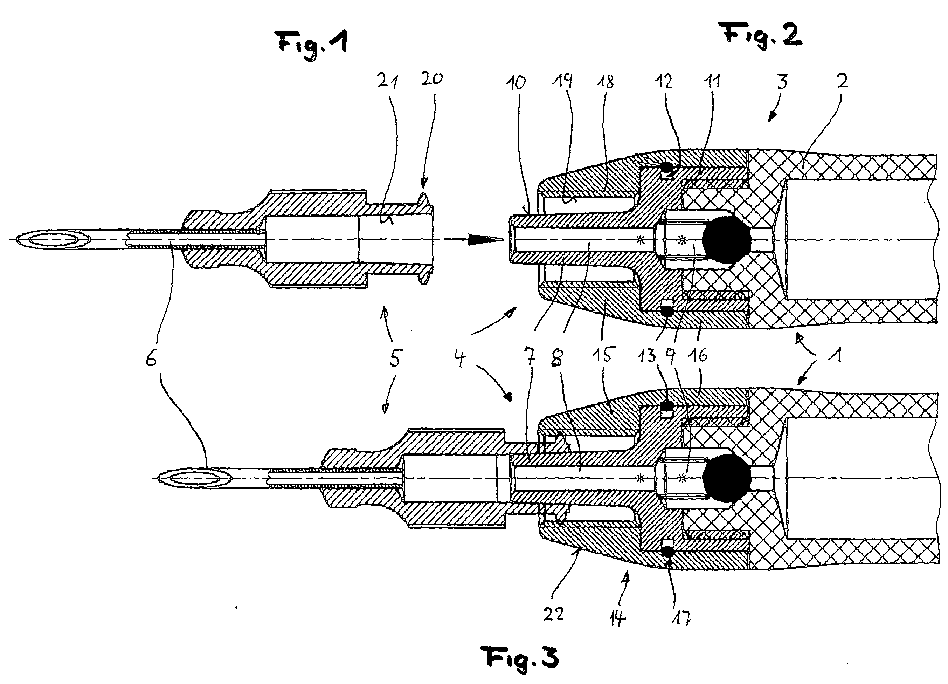

- Fig.1:: eine mit einem Luer-Lock-Ansatz versehene Kanüle,
- Fig.2:: den vorderen Teil eines Spritzenzylinders einer mit einem Luer-Lock-Ansatz versehenen Veterinärspritze sowie
- Fig.3:: den vorderen Teil des Spritzenzylinders mit aufgesetzter Kanüle.

Die in den Figuren nicht vollständig, sondern nur mit dem ausspritzseitigen Ende 1 eines Spritzenzylinders 2 dargestellte Veterinärspritze 3 weist einen Ansatz 4 für das darin einzusetzende Ansatzende 5 einer Kanüle 6 auf.

Der Ansatz 4 ist im wesentlichen zweiteilig ausgebildet und besteht zum ersten aus einem fest am Spritzenzylinder 2 befestigten Hohlzapfen 7, der einen Ausspritzkanal 8 aufweist, der über eine nicht näher zu beschreibende Ventilvorrichtung 9 mit dem Innenraum des Spritzenzylinders 2 verbunden ist. Dabei weist der Hohlzapfen 7 einen dem Ansatzende 5 der Kanüle 6 zugewandten, in seinem Außenwandbereich leicht konisch ausgebildeten Aufsteckbereich 10 auf und einen, dem Spritzenzylinder 2 zugewandten und zur Befestigung auf diesem vorgesehenen und einen größeren Durchmesser aufweisenden Sockel 11 auf. Dieser Sockel 11 ist zylindrisch ausgebildet und weist auf seiner Außenwand eine umfangsmäßig verlaufende Nut 12 auf, in die ein Sprengring 13 eingelegt ist.

Dieser so ausgebildete Hohlzapfen 7 ist umgeben von dem zweiten Teil des Ansatzes 4, nämlich dem Hohlzylinder 14, der einen vorderen und einen hinteren Abschnitt 15 bzw. 16 aufweist, die sich dadurch unterscheiden, daß der hintere Abschnitt 16 einen Innendurchmesser aufweist, der dem Außendurchmesser des Sockels 11 entspricht und auf diesem drehbar gleitend aufliegt. Dabei weist die Innenwand dieses hinteren Abschnittes 16 eine der Nut 12 des Sockels 11 genau gegen-überliegende Gegennut 17 auf, in die ebenfalls der Sprengring 13 einsteht. Hierdurch wird gewährleistet, daß der Hohlzylinder 14, obwohl auf dem Sockel 11 drehbar gelagert, nicht von diesem abgezogen werden kann.

Der vordere Abschnitt 15 des Hohlzylinders 14 weist dagegen einen geringeren Innendurchmesser auf, der jedoch größer ist als der größte Durchmesser des konisch ausgebildeten Aufsteckbereiches 10 des Hohlzapfens 7. Insbesondere weist die zylindrische Innenwand 18 ein Gewinde 19 auf, in das ein am Ansatzende 5 der Kanüle 6 vorgesehenes Gegengewinde 20 einschraubbar ist. Das Ansatzende 5 der Kanüle 6 weist im übrigen eine dem konisch ausgebildeten Aufsteckbereich 10 des Hohlzapfens 7 angepaßten konischen Innenwandbereich 21 auf.

Durch die zweiteilige Ausbildung des Ansatzes 4 ist es möglich, den Hohlzylinder 14 drehbar auf dem Hohlzapfen 7 bzw. auf dessen Sockel 11 anzuordnen. Aufgrund der Drehbarkeit des Hohlzylinders 14 muß nun nicht mehr die Kanüle 6 mit ihrem Gegengewinde 20 in das Gewinde 19 des Hohlzylinders 14 hinein gedreht werden, vielmehr genügt es, wenn die Kanüle mit ihrem Ansatzende 5 auf den konisch ausgebildeten Aufsteckbereich 10 des Hohlzapfens 7 aufgesetzt und mit dem Gegengewinde 20 in Anschlag mit dem Gewinde 19 des Hohlzylinders 14 gebracht wird, sodann wird durch Drehen des Hohlzylinders 14 die Kanüle 6 auf den Hohlzapfen 7 so weit aufgezogen, bis der konisch ausgebildete Aufsteckbereich 10 des Hohlzapfens 7 dicht an dem Innenwandbereich 21 des Ansatzendes 5 der Kanüle 6 anliegt und damit eine dichte Verbindung zwischen Kanüle 6 einerseits und dem Ansatz 4 der Veterinärspritze 3 hergestellt ist.

Um das Betätigen des Hohlzylinders 14 zu erleichtern, ist seine Außenfläche 22 besonders ausgebildet, so kann sie beispielsweise profilierte Grifflächen aufweisen oder auch mit Griffmulden versehen sein.

## Patentansprüche

1. Veterinärspritze mit einem am ausspritzseitigen Ende (1) eines Spritzenzylinders (2) vorgesehenen Ansatz (4) für Kanülen nach Luer-Lock, wobei der Ansatz (4) in seinem vorderen, der aufzusetzenden Kanüle (6) zugewandten Bereich als Hohlzylinder (14) ausgebildet ist, dessen Längsachse mit der Längsachse des Spritzenzylinders (2) der Veterinärspritze (3) fluchtet, und der auf seiner Innenwand (18) ein Gewinde (19) aufweist, das zur Aufnahme eines Ansatzendes (5) einer Kanüle (6) mit entsprechend ausgebildetem Gegengewinde (20) vorgesehen ist, und wobei in dem Innenraum des Hohlzylinders (14) ein, einen gegenüber dem Durchmesser des Innenraumes des Hohlzylinders (14) geringeren Außendurchmesser aufweisender Hohlzapfen (7) angeordnet ist, der den Ausspritzkanal (8) für das Medikament aufweist und in das Ansatzende (5) der aufgesetzten Kanüle (6) einsteht, **dadurch gekennzeichnet, daß** der Hohlzylinder (14) gegenüber dem Hohlzapfen (7) um seine Längsachse drehbar ist.

2. Veterinärspritze nach Anspruch 1, **dadurch gekennzeichnet, daß** der Hohlzapfen (7) mit einem Sockel (11) am Ende (1) des Spritzenzylinders (2) befestigt ist, der zylindrisch ausgebildet ist und auf seiner Außenwand eine über deren Umfang verlaufende Nut (12) aufweist, in die ein Sprengring (13) eingesetzt ist, und daß der Hohlzylinder (14) in Richtung zum Spritzenzylinder (2) verlängert ist und mit einer glattflächigen Innenwand auf der Außenwand des Sockels (11) des Hohlzapfens (7) aufliegt und die Innenwand eine der Nut (12) in der Außenwand des Hohlzapfens (14) genau gegenüberliegende Gegennut (17) aufweist, in die der Sprengring (13) ebenfalls einsteht.

3. Veterinärspritze nach Anspruch 2, **dadurch gekennzeichnet, daß** der Sockel (11) des Hohlzapfens (7) einerseits und der Hohlzylinder (14) in seinem verlängerten Bereich andererseits größere Außen- bzw. Innendurchmesser aufweisen, als in dem für den Ansatz der Kanüle (6) vorgesehenen Bereich.

4. Veterinärspritze nach Anspruch 3, **dadurch gekennzeichnet, daß** die Außenwand des Hohlzylinders (14) in dem für den Ansatz der Kanüle (6) vorgesehenen Bereich kegelförmig ausgebildet ist.

5. Veterinärspritze nach einem der vorstehenden Ansprüche, **dadurch gekennzeichnet, daß** der Hohlzylinder (14) eine Außenfläche (22) aufweist, die profilierte Griffflächen trägt.

6. Veterinärspritze nach einem der vorstehenden Ansprüche, **dadurch gekennzeichnet, daß** der Hohlzylinder (14) eine Außenfläche (22) aufweist, die mit Griffmulden versehen ist.
